# EUROPEAN PATENT SPECIFICATION

(11) **EP 1 259 248 B1**
(45) Date of publication and mention of the grant of the patent: **15.12.2004**
(21) Application number: 01913267.9
(22) Date of filing: 02.03.2001
(51) Int. Cl.: A61K 38/02, A61K 39/395, A61P 35/00

(54) **METHODS FOR TREATING CANCERS EXPRESSING VASCULAR ENDOTHELIAL GROWTH FACTOR D**
METHODE ZUR BEHANDLUNG VON TUMOREN WELCHE DEN VASKULÄREN ENDOTHELIALEN WACHSTUMSFAKTOR D EXPRIMIEREN
PROCEDES DE TRAITEMENT DE CANCERS EXPRIMANT LE FACTEUR D DE CROISSANCE ENDOTHELIALE VASCULAIRE

(30) Priority: 02.03.2000 US 186361 P
(43) Date of publication of application: 27.11.2002
(73) Proprietor: LUDWIG INSTITUTE FOR CANCER RESEARCH, New York, New York 10158 (US)
(72) Inventor: ACHEN, Marc, Ludwig Institute for Cancer Research, Parkville,VIC 3050 (AU); STACKER, Steven, Ludwig Inst. for Cancer Research, Parkville, VIC 3050 (AU)
(74) Representative: Harrison Goddard Foote
(86) International application number: PCT/US2001/006791
(87) International publication number: WO 2001/064235

(56) References cited:
- EP-A- 0 935 001
- WO-A-98/07832
- WO-A-99/33485
- WO-A2-00/37025
- WO-A2-00/77190
- FARNEBO FILIP ET AL: "Restricted expression pattern of vegf-d in the adult and fetal mouse: High expression in the embryonic lung." BIOCHEMICAL AND BIOPHYSICAL RESEARCH COMMUNICATIONS, vol. 257, no. 3, 21 April 1999 (1999-04-21), pages 891-894, XP001135296 ISSN: 0006-291X
- ACHEN M G ET AL: "VASCULAR ENDOTHELIAL GROWTH FACTOR D (VEGF-D) IS A LIGAND FOR THE TYROSINE KINASES VEGF RECEPOR 2 (GLK1) AND VEGF RECEPTOR 3 (FLT4)" PROCEEDINGS OF THE NATIONAL ACADEMY OF SCIENCES OF USA, NATIONAL ACADEMY OF SCIENCE. WASHINGTON, US, vol. 95, January 1998 (1998-01), pages 548-553, XP002905751 ISSN: 0027-8424
- O-CHAROENRAT ET AL.: 'Vascular endothelial growth factor family members are differentially regulated by c-erbB signaling in head and neck squamous carcinoma cells' CLINICAL & EXPERIMENTAL METASTASIS vol. 18, 2000, pages 155 - 161, XP002940801
- STACKER ET AL.: 'VEGF-D promotes the metastatic spread of tumour cells via the lymphatics' NATURE MEDICINE vol. 7, no. 2, February 2001, pages 186 - 191, XP002940802
- ACHEN ET AL.: 'Localization of vascular endothelial growth factor-D in malignant melanoma suggests a role in tumour angiogenesis' JOURNAL OF PATHOLOGY vol. 193, 2001, pages 147 - 154, XP002940803

## Description

### BACKGROUND OF THE INVENTION

The invention generally relates to medicaments for treating and alleviating various cancers.

The two major components of the mammalian vascular system are the endothelial and smooth muscle cells. The endothelial cells form the lining of the inner surface of all blood vessels and lymphatic vessels in the mammal. The formation of new blood vessels can occur by two different processes, vasculogenesis or angiogenesis (for review *see* Risau, W., *Nature* **386**: 671-674, 1997). Vasculogenesis is characterized by the *in situ* differentiation of endothelial cell precursors to mature endothelial cells and association of these cells to form vessels, such as occurs in the formation of the primary vascular plexus in the early embryo. In contrast, angiogenesis, the formation of blood vessels by growth and branching of pre-existing vessels, is important in latter embryogenesis and is responsible for the blood vessel growth which occurs in the adult. Angiogenesis is a physiologically complex process involving proliferation of endothelial cells, degradation of extracellular matrix, branching of vessels and subsequent cell adhesion events. In the adult, angiogenesis is tightly controlled and limited under normal circumstances to the female reproductive system. However angiogenesis can be switched on in response to tissue damage. Importantly solid tumors are able to induce angiogenesis in surrounding tissue, thus sustaining tumor growth and facilitating the formation of metastases (Folkman, J., *Nature Med*. **1**: 27-31, 1995). The molecular mechanisms underlying the complex angiogenic processes are far from being understood.

Angiogenesis is also involved in a number of pathologic conditions, where it plays a role or is involved directly in different sequelae of the disease. Some examples include neovascularization associated with various liver diseases, neovascular sequelae of diabetes, neovascular sequelae to hypertension, neovascularization in post-trauma, neovascularization due to head trauma, neovascularization in chronic liver infection (e.g. chronic hepatitis), neovascularization due to heat or cold trauma, dysfunction related to excess of hormone, creation of hemangiomas and restenosis following angioplasty.

Because of the crucial role of angiogenesis in so many physiological and pathological processes, factors involved in the control of angiogenesis have been intensively investigated. A number of growth factors have been shown to be involved in the regulation of angiogenesis; these include fibroblast growth factors (FGFs), platelet-derived growth factor (PDGF), transforming growth factor alpha (TGFα), and hepatocyte growth factor (HGF). See for example Folkman *et al*., *J*. *Biol*. *Chem*., **267**: 10931-10934, 1992 for a review.

It has been suggested that a particular family of endothelial cell-specific growth factors, the vascular endothelial growth factors (VEGFs), and their corresponding receptors is primarily responsible for stimulation of endothelial cell growth and differentiation, and for certain functions of the differentiated cells. These factors are members of the PDGF/VEGF family, and appear to act primarily via endothelial receptor tyrosine kinases (RTKs). The PDGF/VEGF family of growth factors belongs to the cystine-knot superfamily of growth factors, which also includes the neurotrophins and transforming growth factor-β.

Eight different proteins have been identified in the PDGF/VEGF family, namely two PDGFs (A and B), VEGF and five members that are closely related to VEGF. The five members closely related to VEGF are: VEGF-B, described in International Patent Application PCT/US96/02957 (WO 96/26736) and in U.S. Patents 5,840,693 and 5,607,918 by Ludwig Institute for Cancer Research and The University of Helsinki; VEGF-C or VEGF2, described in Joukov *et al*., *EMBO J*., **15**: 290-298, 1996, Lee *et al*., *Proc. Natl*. *Acad*. *Sci*. *USA*, **93**: 1988-1992, 1996, and U.S. Patents 5,932,540 and 5,935,540 by Human Genome Sciences, Inc; VEGF-D, described in International Patent Application No. PCT/US97/14696 (WO 98/07832), and Achen *et al*., *Proc*. *Natl*. *Acad. Sci*. *USA*, **95**: 548-553, 1998; the placenta growth factor (PlGF), described in Maglione *et al*., Proc. Natl. Acad. Sci. USA, **88**: 9267-9271, 1991; and VEGF3, described in International Patent Application No. PCT/US95/07283 (WO 96/39421), by Human Genome Sciences, Inc. Each VEGF family member has between 30% and 45% amino acid sequence identity with VEGF. The VEGF family members share a VEGF homology domain which contains the six cysteine residues which form the cystine-knot motif. Functional characteristics of the VEGF family include varying degrees of mitogenicity for endothelial cells, induction of vascular permeability and angiogenic and lymphangiogenic properties.

Vascular endothelial growth factor (VEGF) is a homodimeric glycoprotein that has been isolated from several sources. Alterative mRNA splicing of a single VEGF gene gives rise to five isoforms of VEGF. VEGF shows highly specific mitogenic activity for endothelial cells. VEGF has important regulatory functions in the formation of new blood vessels during embryonic vasculogehesis and in angiogenesis during adult life (Carmeliet *et al*., *Nature*, **380**: 435-439, 1996; Ferrara *et al*., *Nature*, **380**: 439-442, 1996; reviewed in Ferrara and Davis-Smyth, *Endocrine Rev*., **18**: 4-25, 1997). The significance of the role played by VEGF has been demonstrated in studies showing that inactivation of a single VEGF allele results in embryonic lethality due to failed development of the vasculature (Carmeliet *et al*., *Nature*, **380**: 435-439, 1996; Ferrara *et al*., *Nature*, **380**: 439-442, 1996). The isolation and properties of VEGF have been reviewed; see Ferrara *et al*., *J*. *Cellular Biochem*., **47**: 211-218, 1991 and Connolly, *J*. *Cellular Biochem*., **47**: 219-223, 1991.

In addition VEGF has strong chemoattractant activity towards monocytes, can induce the plasminogen activator and the plasminogen activator inhibitor in endothelial cells, and can also induce microvascular permeability. Because of the latter activity, it is sometimes referred to as vascular permeability factor (VPF). VEGF is also chemotactic for certain hematopoetic cells. Recent literature indicates that VEGF blocks maturation of dendritic cells and thereby reduces the effectiveness of the immune response to tumors (many tumors secrete VEGF) (Gabrilovich *et al*., *Blood* **92**: 4150-4166, 1998; Gabrilovich *et al*., *Clinical Cancer Research* **5**: 2963-2970, 1999).

VEGF-B has similar angiogenic and other properties to those of VEGF, but is distributed and expressed in tissues differently from VEGF. In particular, VEGF-B is very strongly expressed in heart, and only weakly in lung, whereas the reverse is the case for VEGF. This suggests that VEGF and VEGF-B, despite the fact that they are co-expressed in many tissues, may have functional differences.

VEGF-B was isolated using a yeast co-hybrid interaction trap screening technique by screening for cellular proteins which might interact with cellular retinoic acid-binding protein type I (CRABP-I). Its isolation and characteristics are described in detail in PCT/US96/02957 (WO 96/26736), in U.S. Patents 5,840,693 and 5,607,918 by Ludwig Institute for Cancer Research and The University of Helsinki and in Olofsson *et al*., *Proc*. *Natl*. *Acad*. *Sci*. *USA*, **93**: 2576-2581, 1996.

VEGF-C was isolated from conditioned media of the PC-3 prostate adenocarcinoma cell line (CRL1435) by screening for ability of the medium to produce tyrosine phosphorylation of the endothelial cell-specific receptor tyrosine kinase VEGFR-3 (Flt4), using cells transfected to express VEGFR-3. VEGF-C was purified using affinity chromatography with recombinant VEGFR-3, and was cloned from a PC-3 cDNA library. Its isolation and characteristics are described in detail in Joukov *et al*., *EMBO J*., **15**: 290-298, 1996.

VEGF-D was isolated from a human breast cDNA library, commercially available from Clontech, by screening with an expressed sequence tag obtained from a human cDNA library designated "Soares Breast 3NbHBst" as a hybridization probe (Achen *et al*., *Proc*. *Natl*. *Acad*. *Sci*. *USA*, **95**: 548-553, 1998). Its isolation and characteristics are described in detail in International Patent Application No. PCT/US97/14696 (WO98/07832).

In PCT/US97/14696, the isolation of a biologically active fragment of VEGF-D, designated VEGF-DΔNΔC, is also described. This fragment consists of VEGF-D amino acid residues 93 to 201 linked to the affinity tag peptide FLAG®. The entire disclosure of the International Patent Application PCT/US97/14696 (WO 98/07832) is incorporated herein by reference.

The VEGF-D gene is broadly expressed in the adult human, but is certainly not ubiquitously expressed. VEGF-D is strongly expressed in heart, lung and skeletal muscle. Intermediate levels of VEGF-D are expressed in spleen, ovary, small intestine and colon, and a lower expression occurs in kidney, pancreas, thymus, prostate and testis. No VEGF-D mRNA was detected in RNA from brain, placenta, liver or peripheral blood leukocytes.

PlGF was isolated from a term placenta cDNA library. Its isolation and characteristics are described in detail in Maglione *et al*., *Proc*. *Natl*. *Acad*. *Sci*. *USA*, **88:** 9267-9271, 1991. Presently its biological function is not well understood.

VEGF3 was isolated from a cDNA library derived from colon tissue. VEGF3 is stated to have about 36% identity and 66% similarity to VEGF. The method of isolation of the gene encoding VEGF3 is unclear and no characterization of the biological activity is disclosed.

Similarity between two proteins is determined by comparing the amino acid sequence and conserved amino acid substitutions of one of the proteins to the sequence of the second protein, whereas identity is determined without including the conserved amino acid substitutions.

A major function of the lymphatic system is to provide fluid return from tissues and to transport many extravascular substances back to the blood. In addition, during the process of maturation, lymphocytes leave the blood, migrate through lymphoid organs and other tissues, and enter the lymphatic vessels, and return to the blood through the thoracic duct. Specialized venules, high endothelial venules (HEVs), bind lymphocytes again and cause their extravasation into tissues. The lymphatic vessels, and especially the lymph nodes, thus play an important role in immunology and in the development of metastasis of different tumors. Unlike blood vessels, the embryonic origin of the lymphatic system is not as clear and at least three different theories exist as to its origin. Lymphatic vessels are difficult to identify due to the absence of known specific markers available for them.

Lymphatic vessels are most commonly studied with the aid of lymphography. In lymphography, X-ray contrast medium is injected directly into a lymphatic vessel. The contrast medium gets distributed along the efferent drainage vessels of the lymphatic system and is collected in the lymph nodes. The contrast medium can stay for up to half a year in the lymph nodes, during which time X-ray analyses allow the follow-up of lymph node size and architecture. This diagnostic is especially important in cancer patients with metastases in the lymph nodes and in lymphatic malignancies, such as lymphoma. However, improved materials and methods for imaging lymphatic tissues are needed in the art.

As noted above, the PDGF/VEGF family members act primarily by binding to receptor tyrosine kinases. In general, receptor tyrosine kinases are glycoproteins, which consist of an extracellular domain capable of binding a specific growth factor(s), a transmembrane domain, which is usually an alpha-helical portion of the protein, a juxtamembrane domain, which is where the receptor may be regulated by, e.g., protein phosphorylation, a tyrosine kinase domain, which is the enzymatic component of the receptor and a carboxy-terminal tail, which in many receptors is involved in recognition and binding of the substrates for the tyrosine kinase.

Five endothelial cell-specific receptor tyrosine kinases have been identified, namely VEGFR-1 (Flt-1), VEGFR-2 (KDR/Flk-1), VEGFR-3 (Flt4), Tie and Tek/Tie-2. These receptors differ in their specificity and affinity. All of these have the intrinsic tyrosine kinase activity which is necessary for signal transduction.

The only receptor tyrosine kinases known to bind VEGFs are VEGFR-1, VEGFR-2 and VEGFR-3. VEGFR-1 and VEGFR-2 bind VEGF with high affinity, and VEGFR-1 also binds VEGF-B and PlGF. VEGF-C has been shown to be the ligand for VEGFR-3, and it also activates VEGFR-2 (Joukov *et al*., *The EMBO Journal*, **15**: 290-298, 1996). VEGF-D binds to both VEGFR-2 and VEGFR-3 (Achen *et al*., *Proc*. *Natl*. *Acad*. *Sci*. *USA*, **95**: 548-553, 1998). A ligand for Tek/Tie-2 has been described in International Patent Application No. PCT/US95/12935 (WO 96/11269) by Regeneron Pharmaceuticals, Inc. The ligand for Tie has not yet been identified.

Recently, a novel 130-135 kDa VEGF isoform specific receptor has been purified and cloned (Soker *et al*., *Cell*, **92**: 735-745, 1998). The VEGF receptor was found to specifically bind the VEGF₁₆₅ isoform via the exon 7 encoded sequence, which shows weak affinity for heparin (Soker *et al*., *Cell*, **92**: 735-745, 1998). Surprisingly, the receptor was shown to be identical to human neuropilin-1 (NP-1), a receptor involved in early stage neuromorphogenesis. PlGF-2 also appears to interact with NP-1 (Migdal *et al*., *J*. *Biol*. *Chem*., **273**: 22272-22278, 1998).

VEGFR-1, VEGFR-2 and VEGFR-3 are expressed differently by endothelial cells. Generally, both VEGFR-1 and VEGFR-2 are expressed in blood vessel endothelia (Oelrichs *et al*., *Oncogene*, **8**: 11-18, 1992; Kaipainen *et al*., *J. Exp*. *Med*., **178**: 2077-2088, 1993; Dumont *et al*., *Dev*. *Dyn*., **203**: 80-92, 1995; Fong *et al*., *Dev*. *Dyn*., **207**: 1-10, 1996) and VEGFR-3 is mostly expressed in the lymphatic endothelium of adult tissues (Kaipainen *et al*., *Proc*. *Natl*. *Acad*. *Sci*. *USA*, **9**: 3566-3570, 1995) VEGFR-3 is also expressed in the blood vasculature surrounding tumors.

Although VEGFR-1 is mainly expressed in endothelial cells during development, it can also be found in hematopoetic precursor cells during early stages of embryogenesis (Fong *et al*., *Nature*, **376**: 66-70, 1995). In adults, monocytes and macrophages also express this receptor (Barleon *et al*., *Blood*, **87**: 3336-3343, 1995). In embryos, VEGFR-1 is expressed by most, if not all, vessels (Breier *et al*., *Dev*. *Dyn*., **204**: 228-239, 1995; Fong *et al*., *Dev*. *Dyn*., **207**: 1-10, 1996).

The receptor VEGFR-3 is widely expressed on endothelial cells during early embryonic development but as embryogenesis proceeds becomes restricted to venous endothelium and then to the lymphatic endothelium (Kaipainen *et al*., *Cancer Res*., **54**: 6571-6577, 1994; Kaipainen *et al*., *Proc*. *Natl. Acad*. *Sci*. *USA*, **92**: 3566-3570, 1995). VEGFR-3 is expressed on lymphatic endothelial cells in adult tissues. This receptor is essential for vascular development during embryogenesis.

The essential, specific role in vasculogenesis, angiogenesis and/or lymphangiogenesis of VEGFR-1, VEGFR-2, VEGFR-3, Tie and Tek/Tie-2 has been demonstrated by targeted mutations inactivating these receptors in mouse embryos. Disruption of the VEGFR genes results in aberrant development of the vasculature leading to embryonic lethality around midgestation. Analysis of embryos carrying a completely inactivated VEGFR-1 gene suggests that this receptor is required for functional organization of the endothelium (Fong *et al*., *Nature*, **376**: 66-70, 1995). However, deletion of the intracellular tyrosine kinase domain of VEGFR-1 generates viable mice with a normal vasculature (Hiratsuka *et al*., *Proc. Natl. Acad. Sci*. *USA,* **95**: 9349-9354, 1998). The reasons underlying these differences remain to be explained but suggest that receptor signalling via the tyrosine kinase is not required for the proper function of VEGFR-1. Analysis of homozygous mice with inactivated alleles of VEGFR-2 suggests that this receptor is required for endothelial cell proliferation, hematopoesis and vasculogenesis (Shalaby *et al*., *Nature*, **376**: 62-66, 1995; Shalaby *et al*., *Cell*, **89**: 981-990, 1997). Targeted inactivation of both copies of the VEGFR-3 gene in mice resulted in defective blood vessel formation characterized by abnormally organized large vessels with defective lumens, leading to fluid accumulation in the pericardial cavity and cardiovascular failure at post-coital day 9.5 (Dumont *et al*., *Science*, **282:** 946-949, 1998). On the basis of these findings it has been proposed that VEGFR-3 is required for the maturation of primary vascular networks into larger blood vessels. However, the role of VEGFR-3 in the development of the lymphatic vasculature could not be studied in these mice because the embryos died before the lymphatic system emerged. Nevertheless it is assumed that VEGFR-3 plays a role in development of the lymphatic vasculature and lymphangiogenesis given its specific expression in lymphatic endothelial cells during embryogenesis and adult life. This is supported by the finding that ectopic expression of VEGF-C, a ligand for VEGFR-3, in the skin of transgenic mice, resulted in lymphatic endothelial cell proliferation and vessel enlargement in the dermis. Furthermore this suggests that VEGF-C may have a primary function in lymphatic endothelium, and a secondary function in angiogenesis and permeability regulation which is shared with VEGF (Joukov *et al*., *EMBO J.,* **15**: 290-298, 1996).

In addition, VEGF-like proteins have been identified which are encoded by four different strains of the orf virus. This is the first virus reported to encode a VEGF-like protein. The first two strains are NZ2 and NZ7, and are described in Lyttle *et al*., *J. Virol*., **68:** 84-92, 1994. A third is D1701 and is described in Meyer *et al*., *EMBO J*., **18:** 363-374, 1999. The fourth strain is NZ10 and is described in International Patent Application PCT/US99/25869. It was shown that these viral VEGF-like proteins bind to VEGFR-2 on the endothelium of the host (sheep/goat/human) and this binding is important for development of infection (Meyer *et al*., *EMBO J.,* **18:** 363-374, 1999; Ogawa *et al*. *J*. *Biol*. *Chem*., **273:** 31273-31282, 1988; and International Patent Application PCT/US99/25869). These proteins show amino acid sequence similarity to VEGF and to each other.

The orf virus is a type of species of the parapoxvirus genus which causes a highly contagious pustular dermatitis in sheep and goats and is readily transmittable to humans. The pustular dermatitis induced by orf virus infection is characterized by dilation of blood vessels, swelling of the local area and marked proliferation of endothelial cells lining the blood vessels. These features are seen in all species infected by orf and can result in the formation of a tumor-like growth or nodule due to viral replication in epidermal cells. Generally orf virus infections resolve in a few weeks but severe infections that fail to resolve without surgical intervention are seen in immune impaired individuals.

There is tremendous interest in the development of pharmacological agents which could antagonize the receptor-mediated actions of VEGFs (Martiny-Baron and Marme, *Curr*. *Opin*. *Biotechnol*. **6**: 675-680, 1995). Monoclonal antibodies to VEGF have been shown to suppress tumor growth *in vivo* by inhibiting tumor-associated angiogenesis (Kim *et al*., *Nature* **362**: 841-844, 1993). Similar inhibitory effects on tumor growth have been observed in model systems resulting from expression of either antisense RNA for VEGF (Saleh *et al*., *Cancer Res*. **56**: 393-401, 1996) or a dominant-negative VEGFR-2 mutant (Millauer *et al*., *Nature* **367**: 576-579, 1994).

However, tumor inhibition studies with neutralizing antibodies suggested that other angiogenic factors besides VEGF may be involved (Kim, K. *et al*., *Nature* **362**: 841-844, 1993). Furthermore, the activity of angiogenic factors other than VEGF in malignant melanoma is suggested by the finding that not all melanomas express VEGF (Issa, R. *et al*., *Lab Invest* **79**: 417-425, 1999).

The biological functions of the different members of the VEGF family are currently being elucidated. Of particular interest are the properties of VEGF-D and VEGF-C. These proteins bind to both VEGFR-2 and VEGFR-3 - localized on vascular and lymphatic endothelial cells respectively - and are closely related in primary structure (48% amino acid identity). Both factors are mitogenic for endothelial cells *in vitro*. Recently, VEGF-C was shown to be angiogenic in the mouse cornea model and in the avian chorioallantoic membrane (Cao *et al*., *Proc*. *Natl*. *Acad*. *Sci*. *USA* **95**: 14389-14394, 1998) and was able to induce angiogenesis in the setting of tissue ischemia (Witzenbichler *et al*., *Am*. *J*. *Pathol*. **153**: 381-394, 1998). Furthermore, VEGF-C stimulated lymphangiogenesis in the avian chorioallantoic membrane (Oh *et al*., *Dev*. *Biol*. **188**: 96-109, 1997) and in a transgenic mouse model (Jeltsch *et al*., *Science* **276**: 1423-1425, 1997). VEGF-D was shown to be angiogenic in the rabbit cornea (Marconcini *et al*., *Proc*. *Natl*. *Acad*. *Sci*. *USA* **96**: 9671-9676, 1999). The lymphangiogenic capacity of VEGF-D has not yet been reported, however, given that VEGF-D, like VEGF-C, binds and activates VEGFR-3, a receptor thought to signal for lymphangiogenesis (Taipale *et al*., *Cur*. *Topics Micro*. *Immunol*. **237**: 85-96, 1999), it is highly likely that VEGF-D is lymphangiogenic. VEGF-D and VEGF-C may be of particular importance for the malignancy of tumors, as metastases can spread via either blood vessels or lymphatic vessels; therefore molecules which stimulate angiogenesis or lymphangiogenesis could contribute toward malignancy. This has already been shown to be the case for VEGF. It is noteworthy that VEGF-D gene expression is induced by c-Fos, a transcription factor known to be important for malignancy (Orlandini *et al*., *Proc*. *Natl*. *Acad*. *Sci*. *USA* **93**: 11675-11680, 1996). It is speculated that the mechanism by which c-Fos contributes to malignancy is the upregulation of genes encoding angiogenic factors. Tumor cells deficient in c-fos fail to undergo malignant progression, possibly due to an inability to induce angiogenesis (Saez, E. *et al*., *Cell* **82:** 721-732, 1995). This indicates the existence of an angiogenic factor up-regulated by c-fos during tumor progression.

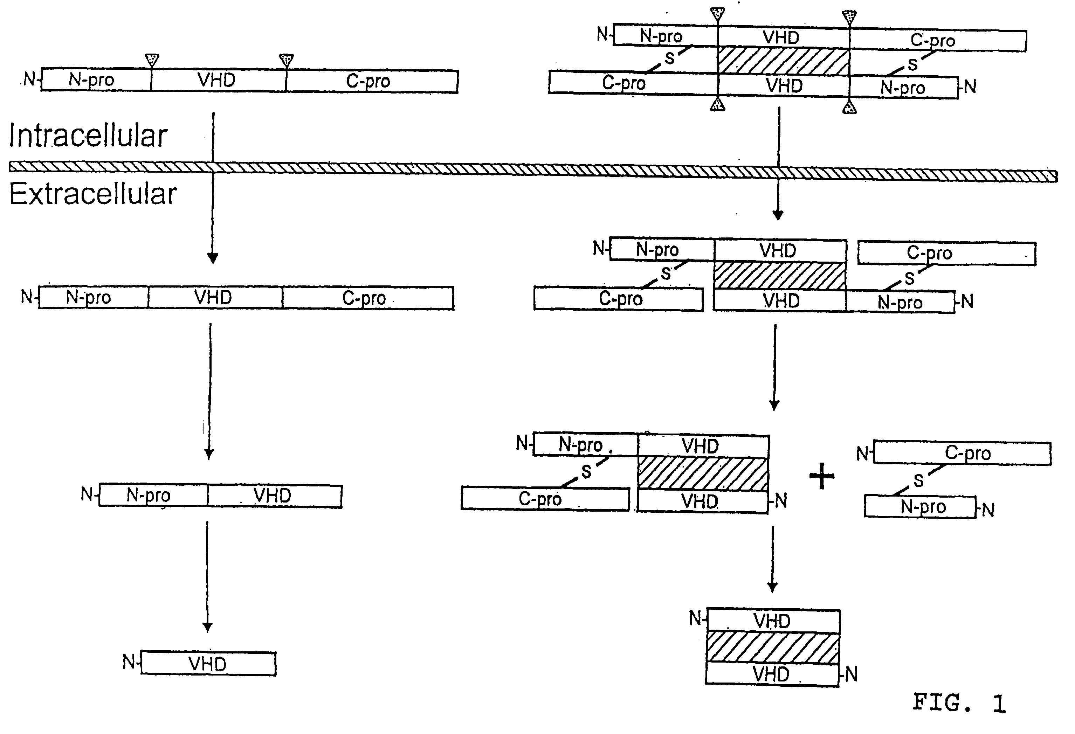

As shown in Figure 1, the predominant intracellular form of VEGF-D is a homodimeric propeptide that consists of the VEGF/PDGF Homology Domain (VHD) and the N- and C-terminal propeptides and has the sequence of SEQ ID NO:2. After secretion, this polypeptide is proteolytically cleaved (Stacker, S.A. *et al*., *J Biol Chem* **274**: 32127-32136, 1999). Proteolytic processing (at positions marked by black arrowheads) gives rise to partially processed forms and a fully processed, mature form which consists of dimers of the VHD. The VHD, which has the sequence of SEQ ID NO:3 (*i*.*e*. residues 93 to 201 of full length VEGF-D), contains the binding sites for both VEGFR-2 and VEGFR-3. The mature form binds both VEGFR-2 and VEGFR-3 with much higher affinity than the unprocessed form (Stacker, S.A. *et al*., *J Biol Chem* **279**: 32127-32136, 1999).

The localization of VEGF-D protein in human cancer has not been studied due to the lack of antibodies specific for the VHD of VEGF-D. Antibodies against the N- or C-terminal propeptides are inappropriate as these regions are cleaved from the bioactive VHD and would localize differently than the VHD (Stacker, S.A. *et al*., *J Biol Chem* **274**: 32127-32136, 1999).

### SUMMARY OF THE INVENTION

The invention generally relates to medicaments for treating and alleviating various cancers.

According to a first aspect, the present invention provides a medicament for treating an organism suffering from a neoplastic disease characterized by the expression of VEGF-D by a tumor selected from breast ductal carcinoma, squamous cell carcinoma, prostate tumors and endometrial cancer.

VEGF-D antagonists may inhibit VEGF-D expression such as with the use of a composition comprising anti-sense nucleic acid or triple-stranded DNA encoding VEGF-D.

VEGF-D antagonists may also inhibit VEGF-D activity such as with the use of compounds comprising antibodies and/or competitive or noncompetitive inhibitors of binding of VEGF-D in both dimer formation and receptor binding. These VEGF-D antagonists include a VEGF-D modified polypeptide, as described below, which has the ability to bind to VEGF-D and prevent binding to the VEGF-D receptors or which has the ability to bind the VEGF-D receptors, but which is unable to stimulate endothelial cell proliferation, differentiation, migration or survival. Small molecule inhibitors to VEGF-D, VEGFR-2 or VEGFR-3 and antibodies directed against VEGF-D, VEGFR-2 or VEGFR-3 may also be used.

It is contemplated that some modified VEGF-D polypeptides will have the ability to bind to VEGF-D receptors on cells including, but not limited to, endothelial cells, connective tissue cells, myofibroblasts and/or mesenchymal cells, but will be unable to stimulate cell proliferation, differentiation, migration, motility or survival or to induce vascular proliferation, connective tissue development or wound healing. These modified polypeptides are expected to be able to act as competitive or non-competitive inhibitors of the VEGF-D polypeptides and growth factors of the PDGF/VEGF family, and to be useful in situations where prevention or reduction of the VEGF-D polypeptide or PDGF/VEGF family growth factor action is desirable. Thus such receptor-binding but non-mitogenic, non-differentiation inducing, non-migration inducing, non-motility inducing, non-survival promoting, non-connective tissue development promoting, non-wound healing or non-vascular proliferation inducing variants of the VEGF-D polypeptide are also within the scope of the invention, and are referred to herein as "receptor-binding but otherwise inactive variant". Because VEGF-D forms a dimer in order to activate its receptors, it is contemplated that one monomer comprises the above-mentioned "receptor-binding but otherwise inactive variant" VEGF-D polypeptide and a second monomer comprises a wild-type VEGF-D or a wild-type growth factor of the PDGF/VEGF family. Thus, these dimers can bind to its corresponding receptor but cannot induce downstream signaling.

It is also contemplated that there are other modified VEGF-D polypeptides that can prevent binding of a wild-type VEGF-D or a wild-type growth factor of the PDGF/VEGF family to its corresponding receptor on cells including, but not limited to, endothelial cells, connective tissue cells (such as fibroblasts), myofibroblasts and/or mesenchymal cells. Thus these dimers will be unable to stimulate endothelial cell proliferation, differentiation, migration, survival, or induce vascular permeability, and/or stimulate proliferation and/or differentiation and/or motility of connective tissue cells, myofibroblasts or mesenchymal cells. These modified polypeptides are expected to be able to act as competitive or non-competitive inhibitors of the VEGF-D growth factor or a growth factor of the PDGF/VEGF family, and to be useful in situations where prevention or reduction of the VEGF-D growth factor or PDGF/VEGF family growth factor action is desirable. Such situations include the tissue remodeling that takes place during invasion of tumor cells into a normal cell population by primary or metastatic tumor formation. Thus such VEGF-D or PDGF/VEGF family growth factor-binding but non-mitogenic, non-differentiation inducing, non-migration inducing, non-motility inducing, non-survival promoting, non-connective tissue promoting, non-wound healing or non-vascular proliferation inducing variants of the VEGF-D growth factor are also within the scope of the invention, and are referred to herein as "the VEGF-D growth factor-dimer forming but otherwise inactive or interfering variants".

Possible modified forms of the VEGF-D polypeptide can be prepared by targeting essential regions of the VEGF-D polypeptide for modification. These essential regions are expected to fall within the strongly-conserved PDGF/VEGF Homology Domain (VDH). In particular, the growth factors of the PDGF/VEGF family, including VEGF, are dimeric, and VEGF, VEGF-B, VEGF-C, VEGF-D, PlGF, PDGF-A and PDGF-B show complete conservation of eight cysteine residues in the VHD (Olofsson *et al*., Proc. Natl. Acad. Sci. USA, 1996 93 2576-2581; Joukov *et al*., EMBO J., 1996 15 290-298). These cysteines are thought to be involved in intra- and inter-molecular disulfide bonding. In addition there are further strongly, but not completely, conserved cysteine residues in the C-terminal domains. Loops 1, 2 and 3 of each VHD subunit, which are formed by intra-molecular disulfide bonding, are involved in binding to the receptors for the PDGF/VEGF family of growth factors (Andersson *et al*., Growth Factors, 1995 12 159-164). Modified polypeptides can readily be tested for their ability to inhibit the biological activity of VEGF-D by routine activity assay procedures such as the endothelial cell proliferation assay.

VEGF-D antagonists useful in the invention may also include molecules comprising polypeptides corresponding to the VEGF-D binding domains of VEGFR-2 (Flk1) or VEGFR-3 (Flt4). For example, the soluble Ig fusion proteins described in Achen *et al*., *Proc. Natl*. *Acad*. *Sci*. *USA,* **95**: 548-553, 1998, which contain the extracellular domains of human VEGFR-2 and human VEGFR-3 and bind to VEGF-DΔNΔC could suitably be used as VEGF-D antagonists.

The medicaments for treating and alleviating various cancers can also occur by targeting a tumor expressing VEGF-D, VEGFR-2 and/or VEGFR-3 for death. This would involve coupling a cytotoxic agent to a polypeptide of the invention, an antibody directed against VEGF-D, VEGFR-2 or VEGFR-3 or a small molecule directed against VEGF-D, VEGFR-2 or VEGFR-3 in order to kill a tumor expressing VEGF-D, VEGFR-2 and/or VEGFR-3. Such cytotoxic agents include, but are not limited to, plant toxins (*e*.*g*. ricin A chain, saporin), bacterial or fungal toxins (*e*.*g*. diphtheria toxin) or radionucleotides (*e*.*g*. 211-Astatine, 212-Bismuth, 90-Yttrium, 131-Iodine, 99-Technitium), alkylating agents (*e*.*g*. chlorambucil), anti-mitotic agents (*e*.*g*. vinca alkaloids), and DNA intercalating agents (*e*.*g*. adriamycin).

The polypeptides, VEGF-D antagonists or antibodies which inhibit the biological activity of VEGF-D also may be employed in combination with a pharmaceutically acceptable non-toxic salt thereof, and a pharmaceutically acceptable solid or liquid carrier or adjuvant. A preferred pharmaceutical composition will inhibit or interfere with a biological activity induced by at least VEGF-D.

Examples of such a carrier or adjuvant include, but are not limited to, saline, buffered saline, Ringer's solution, mineral oil, talc, corn starch, gelatin, lactose, sucrose, microcrystalline cellulose, kaolin, mannitol, dicalcium phosphate, sodium chloride, alginic acid, dextrose, water, glycerol, ethanol, thickeners, stabilizers, suspending agents and combinations thereof. Such compositions may be in the form of solutions, suspensions, tablets, capsules, creams, salves, elixirs, syrups, wafers, ointments or other conventional forms. The formulation can, of course, be adjusted in accordance with known principles to suit the mode of administration. Compositions comprising a peptide of the invention will contain from about 0.1% to 90% by weight of the active compound(s), and most generally from about 10% to 30%.

The dose(s) and route of administration will depend upon the nature of the patient and condition to be treated, and will be at the discretion of the attending physician or veterinarian. Suitable routes include oral, subcutaneous, intramuscular, intraperitoneal or intravenous injection, parenteral, topical application, implants *etc*. For example, an effective amount of a peptide of the invention or antibody is administered to an organism in need thereof in a dose between about 0.1 and 100 mg/kg body weight, and more preferably 1 to 10 mg/kg body weight. For humanized antibodies, which typically exhibit a long circulating half-life, dosing at intervals ranging from daily to every month, and more preferably every week, or every other week, or every third week, are specifically contemplated. Monitoring the progression of the therapy, patient side effects, and circulating antibody levels will provide additional guidance for an optimal dosing regimen. Data from published and ongoing clinical trials for other antibody-based cancer therapeutics (e.g. anti-HER2, anti-EGF receptor) also provide useful dosage regimen guidance. Topical application of VEGF-D may be used in a manner analogous to VEGF.

It will be clearly understood that for the purposes of this specification the phrase "fully processed VEGF-D" means the mature form of VEGF-D polypeptide, *i*.*e*. the VEGF homology domain (VHD), having the sequence of SEQ ID NO:3 which is without the N- and C-terminal propeptides. The phrase "proteolytically processed form of VEGF-D" means a VEGF-D polypeptide without the N- and/or C-terminal propeptide, and the phrase "unprocessed VEGF-D" means a full-length VEGF-D polypeptide having the sequence of SEQ ID NO:2 with both the N- and C-terminal propeptides.

The full length VEGF-D polypeptide havihg the sequence of SEQ ID NO:2 may be optionally linked to the FLAG® peptide. Where the full length VEGF-D polypeptide is linked to FLAG®, the fragment is referred to herein as VEGF-D-FULL-N-FLAG. A. preferred fragment of VEGF-D is the portion of VEGF-D from amino acid residue 93 to amino acid residue 201 (i.e. the VHD (SEQ ID NO:3)), optionally linked to the FLAG® peptide. Where the fragment is linked to FLAG®, the fragment is referred to herein as VEGF-DΔNΔC.

The expression "biological activity of VEGF-D" is to be understood to mean the ability to stimulate one or more of endothelial cell proliferation, differentiation, migration, survival or vascular permeability.

Polypeptides comprising conservative substitutions, insertions, or deletions, but which still retain the biological activity of VEGF-D are clearly to be understood to be within the scope of the invention. Persons skilled in the art will be well aware of methods which can readily be used to generate such polypeptides, for example the use of site-directed mutagenesis, or specific enzymatic cleavage and ligation. The skilled person will also be aware that peptidomimetic compounds or compounds in which one or more amino acid residues are replaced by a non-naturally occurring amino acid or an amino acid analog may retain the required aspects of the biological activity of VEGF-D. Such compounds can readily be made and tested by methods known in the art, and are also within the scope of the invention.

Preferably where amino acid substitution is used, the substitution is conservative, i.e. an amino acid is replaced by one of similar size and with similar charge properties.

As used herein, the term "conservative substitution" denotes the replacement of an amino acid residue by another, biologically similar residue. Examples of conservative substitutions include the substitution of one hydrophobic residue such as isoleucine, valine, leucine, alanine, cysteine, glycine, phenylalanine, proline, tryptophan, tyrosine, norleucine or methionine for another, or the substitution of one polar residue for another, such as the substitution of arginine for lysine, glutamic acid for aspartic acid, or glutamine for asparagine, and the like. Neutral hydrophilic amino acids which can be substituted for one another include asparagine, glutamine, serine and threonine. The term "conservative substitution" also includes the use of a substituted amino acid in place of an unsubstituted parent amino acid.

As such, it should be understood that in the context of the present invention, a conservative substitution is recognized in the art as a substitution of one amino acid for another amino acid that has similar properties. Exemplary conservative substitutions are set out in the following Table A from WO 97/09433.

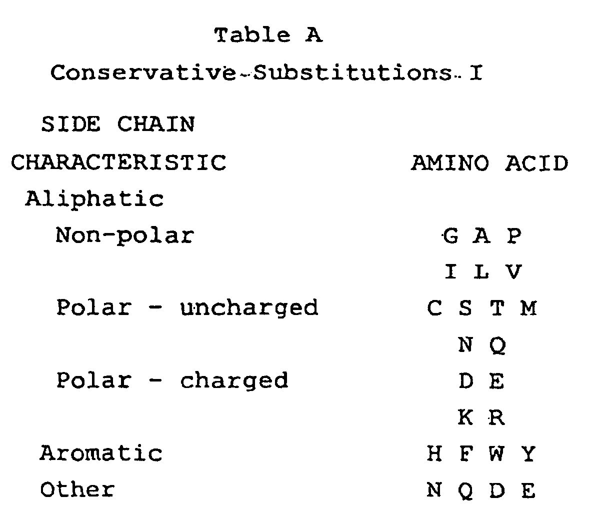

Alternatively, conservative amino acids can be grouped as described in Lehninger, [Biochemistry, Second Edition; Worth Publishers, Inc. NY:NY (1975), pp.71-77] as set out in the following Table B.

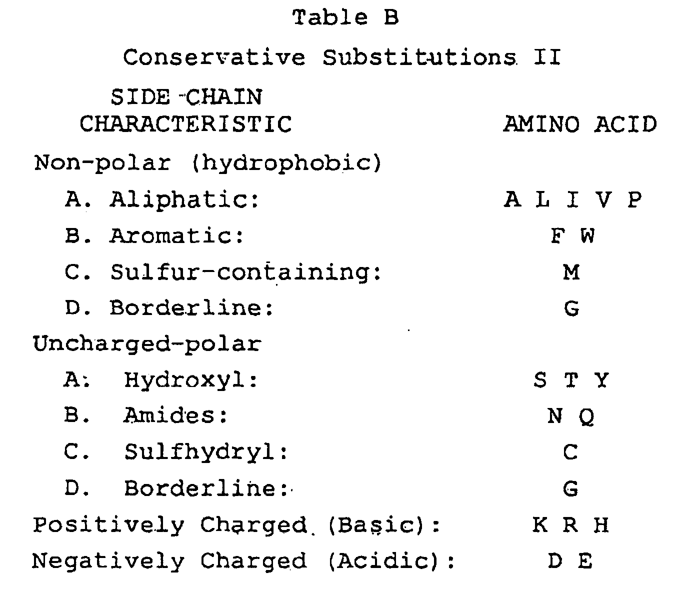

Exemplary conservative substitutions are set out in the following Table C.

**Table C**

| Conservative Substitutions III | |
|---|---|
| Original Residue | Exemplary Substitution |
| Ala (A) | Val, Leu, Ile |
| Arg (R) | Lys, Gln, Asn |
| Asn (N) | Gln, His, Lys, Arg |
| Asp (D) | Glu |
| Cys (C) | Ser |
| Gln (Q) | Asn |
| Glu (E) | Asp |
| His (H) | Asn, Gln, Lys, Arg |
| Ile (I) | Leu, Val, Met, Ala, Phe, |
| Leu (L) | Ile, Val, Met, Ala, Phe |
| Lys (K) | Arg, Gln, Asn |
| Met (M) | Leu, Phe, Ile |
| Phe (F) | Leu, Val, Ile, Ala |
| Pro (P) | Gly |
| Ser (S) | Thr |
| Thr (T) | Ser |
| Trp (W) | Tyr, Phe |
| Tyr (Y) | Trp, Phe, Thr, Ser |
| Val (V) | Ile, Leu, Met, Phe, Ala |

Possible variant forms of the VEGF-D polypeptide which may result from alternative splicing, as are known to occur with VEGF and VEGF-B, and naturally-occurring allelic variants of the nucleic acid sequence encoding VEGF-D are encompassed within the scope of the invention. Allelic variants are well known in the art, and represent alternative forms of a nucleic acid sequence which comprise substitution, deletion or addition of one or more nucleotides, but which do not result in any substantial functional alteration of the encoded polypeptide.

Such variant forms of VEGF-D can be prepared by targeting non-essential regions of the VEGF-D polypeptide for modification. These non-essential regions are expected to fall outside the strongly-conserved regions. In particular, the growth factors of the PDGF/VEGF family, including VEGF, are dimeric, and VEGF, VEGF-B, VEGF-C, VEGF-D, PlGF, PDGF-A and PDGF-B show complete conservation of eight cysteine residues in the N-terminal domains, *i*.*e*. the PDGF/VEGF-like domains (Olofsson *et al*., Proc. Natl. Acad. Sci. USA, 1996 93 2576-2581; Joukov *et al*., EMBO J., 1996 15 290-298). These cysteines are thought to be involved in intra- and inter-molecular disulfide bonding. In addition there are further strongly, but not completely, conserved cysteine residues in the C-terminal domains. Loops 1, 2 and 3 of each VHD subunit, which are formed by intra-molecular disulfide bonding, are involved in binding to the receptors for the PDGF/VEGF family of growth factors (Andersson *et al*., Growth Factors, 1995 12 159-164).

Persons skilled in the art thus are well aware that these cysteine residues should be preserved in any proposed variant form, and that the active sites present in loops 1, 2 and 3 also should be preserved. However, other regions of the molecule can be expected to be of lesser importance for biological function, and therefore offer suitable targets for modification. Modified polypeptides can readily be tested for their ability to show the biological activity of VEGF-D by routine activity assay procedures such as the endothelial cell proliferation assay.

It has been shown that a strong signal for VEGF-D is present in a subset of hematopoetic cells. These cells flood into the peripheral regions of some tumors in a type of inflammatory response. Thus, inhibition of this process would be useful where it is desirable to prevent this inflammatory response.

In accordance with a further aspect of the invention, the invention relates to a medicament for treating an organism, e.g. a mammal, suffering from a neoplastic disease characterized by the expression of VEGF-D by a tumor selected from breast ductal carcinoma, squamous cell carcinoma, prostate cancer or endometrial cancer, comprising administering an effective amount of a VEGF-D antagonist in the vicinity of said tumor to prevent binding of VEGF-D to its corresponding receptor. If desired, a cytotoxic agent may be co-administered with the VEGF-D antagonist. A preferred VEGF-D antagonist is a monoclonal antibody which specifically binds VEGF-D and blocks VEGF-D binding to VEGF Receptor-2 or VEGF Receptor-3, especially an antibody which binds to the VEGF homology domain of VEGF-D.

It will be clearly understood that for the purposes of this specification the word. "comprising" means "including but not limited to". The corresponding meaning applies to the word "comprises".

### BRIEF DESCRIPTION OF THE DRAWINGS

Figure 1 is a schematic representation of VEGF-D processing;
Figure 2 shows the specificity of MAb 4A5 for the VEGF/PDGF Homology Domain (VHD) of human VEGF-D as assessed by Western blot analysis;
Figure 3 shows autoradiographs taken after two days of exposure to mouse 15.5 days post-coital tissue sections hybridized with VEGF-D antisense and sense RNAs;
Figures 4A-4D show the results of analysis of the distribution of VEGF-D mRNA in the post-coital day 15.5 mouse embryo by *in situ* hybridization;
Figures 5A-5F show the localization of VEGF-D in squamous cell carcinoma of the lung;
Figures 6A-6F show the localization of VEGF-D in breast ductal carcinoma *in situ*;
Figure 7 shows the localization of VEGF-D in endometrial adenocarcinoma *in situ*;
Figure 8 shows the results of the analysis of tumors in SCID mice resulting from injection of untransfected parental 293 cells (designated "293") and 293 cells transfected with ah expression vector encoding VEGF-D-FULL-N-FLAG (designated "VEGF-D-293").
Figure 9 shows increased rate of growth in tumors from the VEGF-DΔN cells.
Figure 10 shows a tumor produced by VEGF-DΔN cells.
Figure 11 shows a normal tumor.
Figure 12 shows the decreased rate of growth in tumors from VEGF-DΔNΔC cells.

### DETAILED DESCRIPTION OF PREFERRED EMBODIMENTS

### Example 1 Production of monoclonal antibodies that bind to human VEGF-D

In order to detect the VEGF/PDGF Homology Domain (VHD) rather than the N- and C-terminal propeptides, monoclonal antibodies to the mature form of human VEGF-D (residues 93 to 201 of full-length VEGF-D (SEQ ID NO:2), *i*.*e*. with the N- and C-terminal regions removed) were raised in mice. A DNA fragment encoding residues 93 to 201 was amplified by polymerase chain reaction (PCR) with *Pfu* DNA polymerase, using as template a plasmid comprising full-length human VEGF-D cDNA (SEQ ID NO:1). The amplified DNA fragment, the correctness of which was confirmed by nucleotide sequencing, was then inserted into the expression vector pEFBOSSFLAG (a gift from Dr. Clare McFarlane at the Walter and Eliza Hall Institute for Medical Research (WEHI), Melbourne, Australia) to give rise to a plasmid designated pEFBOSVEGF-DΔNΔC. The pEFBOSSFLAG vector contains DNA encoding the signal sequence for protein secretion from the interleukin-3 (IL-3) gene and the FLAG® octapeptide (Sigma-Aldrich). The FLAG® octapeptide can be recognized by commercially available antibodies such as the M2 monoclonal antibody (Sigma-Aldrich). The VEGF-D PCR fragment was inserted into the vector such that the IL-3 signal sequence was immediately upstream from the FLAG® octapeptide, which was in turn immediately upstream from the truncated VEGF-D sequence. All three sequences were in the same reading frame, so that translation of mRNA resulting from transfection of pEFBOSVEGF-DΔNΔC into mammalian cells would give rise to a protein which would have the IL-3 signal sequence at its N-terminus, followed by the FLAG® octapeptide and the truncated VEGF-D sequence. Cleavage of the signal sequence and subsequent secretion of the protein from the cell would give rise to a VEGF-D polypeptide which is tagged with the FLAG® octapeptide adjacent to the N-terminus. VEGF-DΔNΔC was purified by anti-FLAG® affinity chromatography from the medium of COS cells which had been transiently transfected with the plasmid pEFBOSVEGF-DΔNΔC. (see Example 9 in International Patent Application No. PCT/US97/14696).

Purified VEGF-DΔNΔC was used to immunize female Balb/C mice on day 85 (intraperitoneal), 71 (intraperitoneal) and 4 (intravenous) prior to the harvesting of the spleen cells from the immunized mice and subsequent fusion of these spleen cells to mouse myeloma P3X63Ag8.653 (NS-1) cells. For the first two immunizations, approximately 10 µg of VEGF-DΔNΔC in a 1:1 mixture of PBS and TiterMax adjuvant (#R-1 Research adjuvant; CytRx Corp., Norcross, GA) were injected, whereas for the third immunization 35 µg of VEGF-DΔNΔC in PBS was used.

Monoclonal antibodies to VEGF-DΔNΔC were selected by screening the hybridomas on purified VEGF-DΔNΔC using an enzyme immunoassay. Briefly, 96-well microtiter plates were coated with VEGF-DΔNΔC, and hybridoma supernatants were added and incubated for 2 hours at 4°C, followed by six washes in PBS with 0.02% Tween 20. Incubation with a horse radish peroxidase conjugated anti-mouse Ig (Bio-Rad, Hercules, CA) followed for 1 hour at 4°C. After washing, the assay was developed with an 2,2'-azino-di-(3-ethylbenz-thiazoline sulfonic acid) (ABTS) substrate system (Zymed, San Francisco, CA), and the assay was quantified by reading absorbance at 405 nm in a multiwell plate reader (Flow Laboratories MCC/340, McLean, VA). Six antibodies were selected for further analysis and were subcloned twice by limiting dilution. These antibodies were designated 2F8, 3C10, 4A5, 4E10, 4H4 and 5F12. The isotypes of the antibodies were determined using an Isostrip™ isotyping kit (Boehringer Mannheim, Indianapolis, IN). Antibodies 2F8, 4A5, 4E10 and 5F12 were of the IgG₁ class whereas 4H4 and 3C10 were of the IgM class. All six antibodies contained the kappa light chain.

Hybridoma cell lines were grown in DMEM containing 5% v/v IgG-depleted serum (Gibco BRL, Gaithersburg, MD), 5mM L-glutamine, 50 µg/ml gentamicin and 10 µg/ml recombinant IL-6. Antibodies 2F8, 4A5, 4E10 and 5F12 were purified by affinity chromatography using protein G-Sepharose according to the technique of Darby *et al*., *J*. *Immunol*. *Methods* **159**: 125-129, 1993, and the yield assessed by measuring absorption at 280nm.

### Example 2 Specificity of 4A5

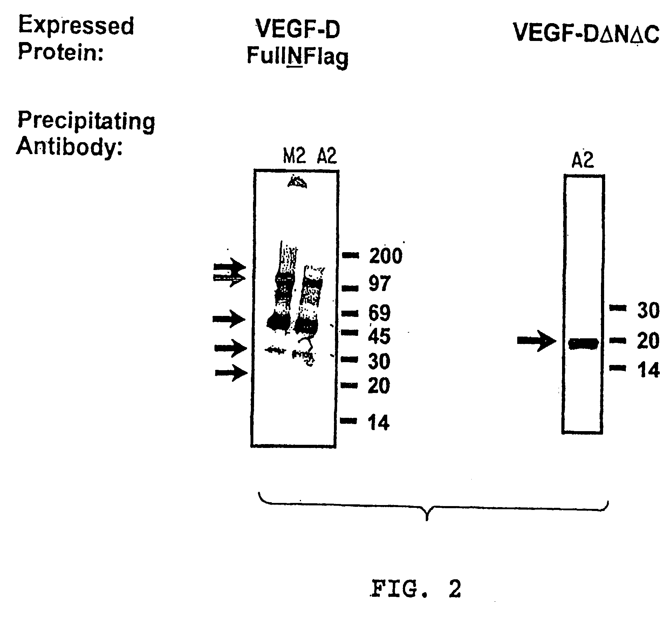

The specificity of MAb 4A5 (renamed VD1) for the VHD of human VEGF-D was assessed by Western blot analysis.. Derivatives of VEGF-D used were VEGF-DΔNΔC, consisting of amino acid residues 93 to 201 of human VEGF-D tagged at the N-terminus with the FLAG® octapeptide (Example 1), VEGF-D-FULL-N-FLAG, consisting of full-length VEGF-D tagged at the N-terminus with FLAG® (Stacker, S.A. *et al*., *J Biol Chem* **274:** 32127-32136, 1999), and VEGF-D-CPRO, consisting of the C-terminal propeptide, from amino acid residues 206 to 354, which was also tagged with FLAG® at the N-terminus. These proteins were expressed in 293-EBNA-1 cells, purified by affinity chromatography with M2 (anti-FLAG®) MAb (IBI/Kodak, New Haven, CT) using the procedure set forth in Achen, M. *et al*., *Proc Natl Acad Sci USA* **95**: 548-553, 1998. Fifty nanograms of purified VEGF-D-FULL-N-FLAG (FN), VEGF-DΔNΔC (ΔΔ), and VEGF-D-CPRO (CP) were analyzed by SDS-PAGE (reducing) and by Western blot using the VD1 MAb and a biotinylated M2 MAb as control (the antibody used for blotting is indicated at the bottom of the panel of Figure 2). SDS-Page and Western blot analyses were carried out as described in Stacker, S.A. *et al*., *J Biol Chem* **274:** 32127-32136, 1999.

As shown in Figure 2, the predominant species in the sample of VEGF-D-FULL-N-FLAG consist of unprocessed VEGF-D (Mr ∼53 K), partially processed VEGF-D containing both the N-terminal propeptide and the VHD (∼31 K), and the N-terminal propeptide (∼10 K) (Stacker, S.A. *et al*., *J Biol Chem* **274:** 32127-32136, 1999), all of which are detected with the M2 MAb as they are tagged with the FLAG® octapeptide (arrows to the left, numbers represent Mr in K and subscripts indicate the sample in which the band is detected). Likewise, VEGF-DΔNΔC (∼21 K) and VEGF-D-CPRO (two bands of ∼31 and ∼29 K which arise due to differential glycosylation) are detected with M2 (arrows to the left) as these polypeptides are also tagged with FLAG®. VD1 detects unprocessed VEGF-D, partially processed VEGF-D and VEGF-DΔNΔC, but not the N-terminal propeptide (∼10 K) in the VEGF-D-FULL-N-FLAG preparation, nor the C-terminal propeptide in the VEGF-D-CPRO sample (∼31 and ∼29 K). Results with VEGF-D-FULL-N-FLAG were analyzed with long (L) and short (S) exposures. The positions of molecular weight markers are shown to the right in Figure 2.

Thus MAb VD1 binds unprocessed VEGF-D, partially processed forms containing the VHD and fully processed VEGF-D, but not the N- or C-terminal propeptides. Furthermore, MAb VD1 was able to immunoprecipitate native human VEGF-DΔNΔC, but not the VHD of human VEGF-C (VEGF-CΔNΔC) (Joukov, V. *et al*., *EMBO J* **16:** 3898-3911, 1997) in an enzyme immunoassay indicating that VD1 is specific for VEGF-D.

### Example 3 In Situ Hybridization Studies of VEGF-D Gene Expression in Mouse Embryos

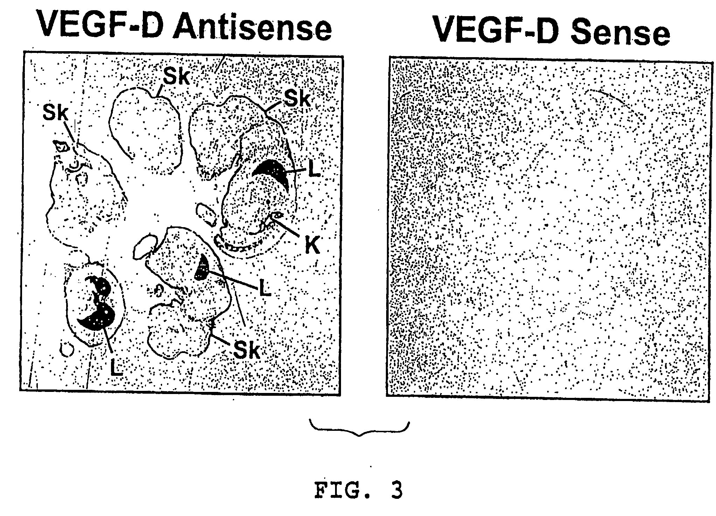

The pattern of VEGF-D gene expression was studied by *in situ* hybridization using a radiolabeled antisense RNA probe corresponding to nucleotides 1 to 340 of the mouse VEGF-D1 cDNA (SEQ ID NO:4). The antisense RNA was synthesized by *in vitro* transcription with T3 RNA polymerase and [³⁵S]UTPαs. Mouse VEGF-D is fully described in International Patent application PCT/US97/14696 (WO 98/07832). This antisense RNA probe was hybridized to paraffin-embedded tissue sections of mouse embryos at post-coital day 15.5. The labeled sections were subjected to autoradiography for 2 days. The resulting autoradiographs for sections hybridized to the antisense RNA and to complementary sense RNA (as negative control) are shown in Figure 3. In Figure 3, "L" denotes lung and "Sk" denotes skin, and the two tissue sections shown are serial sections. Strong signals for VEGF-D mRNA were detected in the developing lung and associated with the skin. No signals were detected using the control sense RNA.

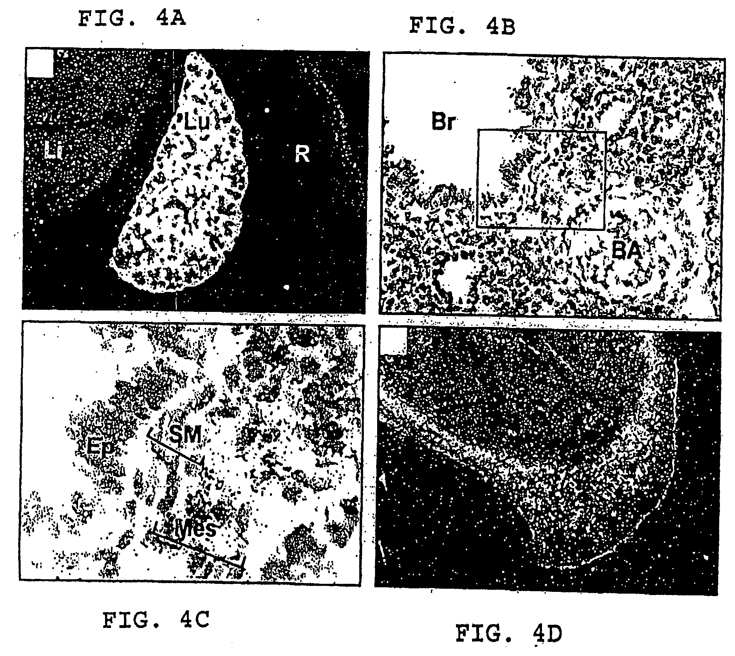

In Figures 4A-4D, sagittal tissue sections were hybridized with the VEGF-D antisense RNA probe and subsequently incubated with photographic emulsion, developed and stained. The magnification for Figures 4A and 4D is x40, for Figure 4B, it is x200 and for Figure 4C, it is x500.

In Figure 4A, the dark field micrograph shows a strong signal for VEGF-D mRNA in lung (Lu). Liver (Li) and ribs (R) are also shown. Figure 4B shows a higher magnification of the lung. This light field micrograph shows a bronchus (Br) and bronchial artery (BA). The black, outline of a rectangle denotes the region of the section shown in Figure 4C but at a higher magnification. Figure 4C shows the epithelial cells of the bronchus (Ep), the developing smooth muscle cells (SM) surrounding the epithelial cell layer and the mesenchymal cells (Mes). The abundance of silver grains associated with mesenchymal cells is apparent. Thus, microscopic analysis reveals that VEGF-D mRNA is abundant in the mesenchymal cells of the developing lung (Figures 4A-4C). In contrast, the epithelial cells of the bronchi and bronchioles are negative, as were the developing smooth muscle cells surrounding the bronchi. The endothelial cells of bronchial arteries are also negative.

In Figure 4D, a dark field micrograph shows a limb bud. A strong signal was located immediately under the skin in a region of tissue rich in fibroblasts and developing melanocytes.

These results indicate that VEGF-D may attract the growth of blood and lymphatic vessels into the developing lung and into the region immediately underneath the skin.

### Example 4 VEGF-D in lung cancer

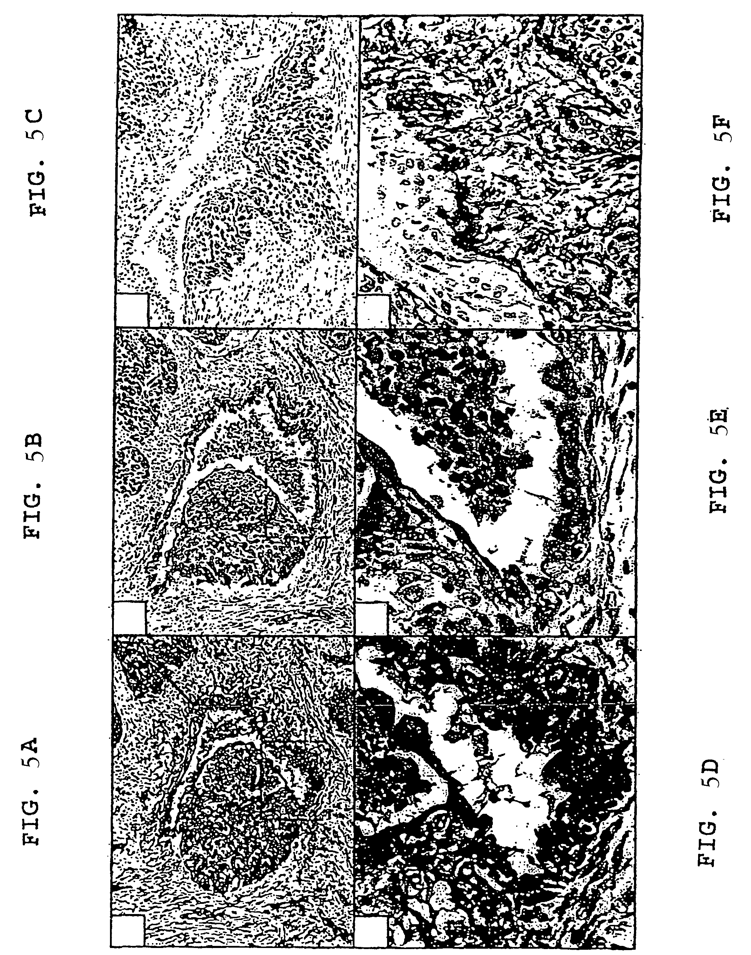

Neoangiogenesis is thought to be a useful prognostic indicator for non-small cell lung carcinoma (NSCLC) (Fontanini, G. *et al*., *Clin Cancer Res*. **3**: 861-865, 1997). Therefore localization of VEGF-D was analyzed in a case of squamous cell carcinoma of the lung by immunohistochemistry (Figures 5A-5F). Immunohistochemistry was conducted using antibodies to alpha-smooth muscle actin (DAKO Corp., Carpinteria, CA) were used to immunostain. The anti-VEGF-D MAb used for immunostaining in Figures 5A and 5D was VD1 (4A5). Figure 5A shows that VEGF-D is detected in tumor cells that form an island at the center of the photomicrograph, in cells lining the adjacent large vessel and in cells within the desmoplastic stroma. The desmoplastic stroma is indicated by a black bracket and the dotted box denotes the region shown in higher power in Figure 5D. The immunopositive cells in the stroma may be myofibroblasts.

Figure 5B shows that VEGFR-2 is detected in cells lining the large vessel. However, these vessels were negative for VEGFR-3 in this tumor. The dotted box denotes the region shown in higher power in Figure 5E. Control staining, of a tissue section from the same case, in which VEGF-D MAb had been preincubated with a 40-fold molar excess of the VHD of human VEGF-D gave no signal (Figure 5C).

As mentioned above, the immunopositive cells in the desmoplatic stroma may be myofibroblasts. Therefore, the desmoplastic stroma was immunostained using MAbs specific for alpha-smooth muscle actin that detect myofibroblasts. As seen in Figure 5F, the stroma stained positive, indicating the presence of myofibroblasts. Secretion of an angiogenic factor by stromal components may serve to amplify the angiogenic stimulus generated by the tumor.

### Example 5 VEGF-D in breast cancer

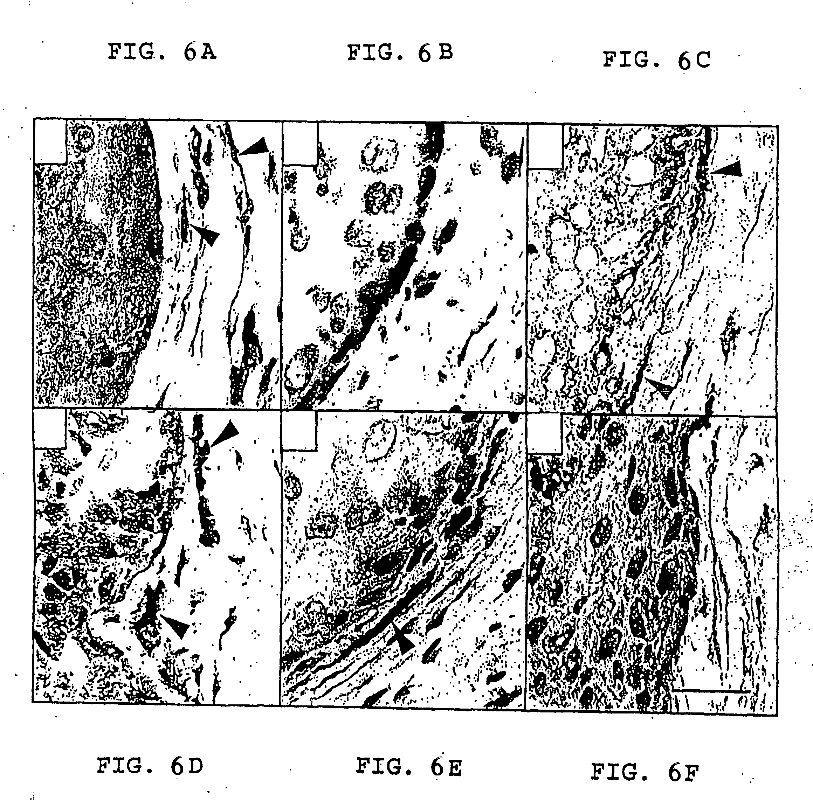

Localization of VEGF-D was also analyzed in breast ductual carcinoma *in situ* by immunohistochemistry, the results of which are shown in Figures 6A-6F. The immunohistochemistry was using MAbs specific for alpha-smooth muscle actin (DAKO Corp., Carpinteria, CA) and the platelet/endothelial adhesion molecule (PECAM) (DAKO Corp., Carpinteria, CA) were also used to immunostain. The anti-VEGF-D MAb used for immunostaining in Figure 6A was VD1 (4A5).

As seen in Figure 6A, VEGF-D was detected in tumor cells in the ducts and in small so-called "necklace" vessels (denoted by black arrowheads) immediately adjacent to the basal lamina of the tumor-filled ducts. The necklace vessels were also positive for VEGFR-2 (Figure 6C), VEGFR-3 (Figure 6D) and PECAM (Figure 6E) as indicated by the black arrowheads. PECAM is a classic marker for endothelium and is also found on platelets and leukocytes. PECAM plays a role in the emigration of leukocytes to inflammatory sites (Muller *et al*., *J. Exp*. *Med*. **178**: 449-460). PECAM antibody staining on the "necklace" vessels helps to confirm that these structures are vessels. The edge of the duct is identified by staining for alpha-smooth muscle actin (Figure 6B) that detects myofibroblasts. Control staining, of a tissue section serial to that shown in Figure 6A, in which VEGF-D MAb had been preincubated with a 40-fold molar excess of the VHD of human VEGF-D gave no signal (Figure 6F). These findings indicate that VEGF-D, secreted by the tumor cells, could activate its receptors on vessels in the vicinity and thereby play a role in attracting the growth of the necklace vessels to their positions very close to the ducts. This could be of importance both for solid tumor growth and metastatic spread.

### Example 6 VEGF-D in endometrial cancer

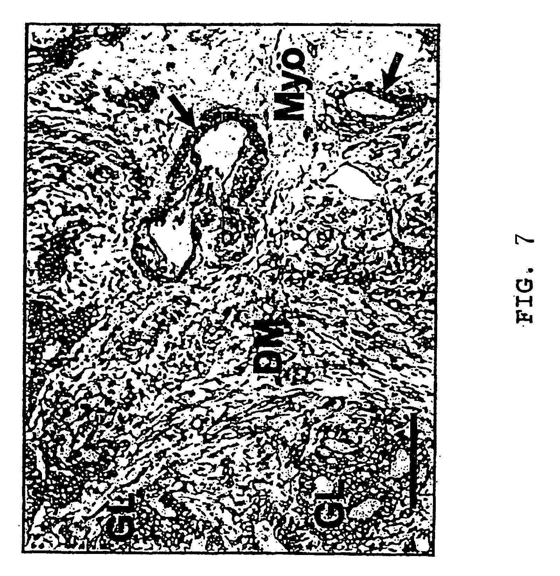

VEGF-D was also detected in endometrial adenocarcinoma (Figure 7). The immunohistochemistry was carried out using the anti-VEGF-D MAb VD1 (4A5). Moderate staining for VEGF-D was seen in the glandular tumor cells (GL), very strong reactivity was seen in the myofibroblastic cells of the desmoplastic stroma (DM) at the advancing invasive edge of the tumor and strong reactivity in the endothelium and walls of adjacent blood vessels (black arrows) in the myometrium (Myo). Interestingly, VEGF-D reactivity was particularly strong in the myofibroblasts of the desmoplastic stroma, indicating that the glandular tumor cells can induce VEGF-D expression in these fibroblasts which would amplify the angiogenic potential of the tumor. As expression of VEGF-D in cells of the desmoplastic stroma was also detected in lung carcinoma (Figure 5A), it may be that a range of tumors can induce VEGF-D in stromal components. This is analogous to the developing lung where the mesenchymal cells, presumably fibroblastic precursors, strongly express the VEGF-D gene. Therefore, signals from both embryonic and tumor tissues can induce expression of VEGF-D in fibroblasts.

### Example 7 Role of VEGF-D in Tumor Development

In order to generate cell lines constitutively over-expressing derivatives of VEGF-D, regions of the human VEGF-D cDNA were inserted into the mammalian expression vector Apex-3 (Evans *et al*, Mol. Immunol., 1995 32 1183-1195). This vector is maintained episomally when transfected into 293-EBNA human embryonal kidney cells. For expression of mature VEGF-D, the region of pEFBOSVEGF-DΔNΔC containing the sequences encoding the IL-3 signal sequence, the FLAG® octapeptide and the mature VEGF-D were inserted into the *Xba*I site of Apex-3 (see Example 9 in International Patent Application PCT/US97/14696 (WO98/07832)). The resulting plasmid was designated pVDApexDΔNΔC (Stacker, S.A. *et al*., *J Biol Chem* **274**: 32127-32136, 1999 and see Example 1 in International Patent Application PCT/US98/27373). A similar construct was made for expression of the unprocessed full-length VEGF-D tagged at the N-terminus with Flag®. In this construct, the DNA encoding the VEGF-D signal sequence for protein secretion was deleted and substituted with DNA encoding the IL-3 signal sequence, followed by the FLAG® octapeptide and two amino acids (Thr-Arg) immediately upstream and in the same reading frame as DNA encoding residues 24-354 of VEGF-D. This construct was designated pVDApexFull-N-Flag (Stacker, S.A. *et al*., *J Biol Chem* **274:** 32127-32136, 1999 and see Example 1 in International Patent Application PCT/US98/27373). These vectors were transfected into cells of the human embryo kidney cell line 293EBNA-1 by the calcium phosphate method or with Fugene® according to the manufacturer's instructions (Roche Molecular Biochemicals, Mannhiem, Germany), and stable transfectants were selected in the presence of 100 µg/ml hygromycin supplemented DMEM. Cell lines expressing high levels of VEGF-D-Full-N-Flag and VEGF-DΔNΔC were subsequently identified by metabolic labeling, immunoprecipitation and Western blot analysis (Stacker, S.A. *et al*., *J*. *Biol Chem* **274:** 32127-32136, 1999 and see Example 1 in International Patent Application PCT/US98/27373).

Six to eight weeks old SCID mice (ARC, Perth, Australia) were injected subcutaneously in the mammary fat pad with 2 x 10⁷ of the transfected 293 cells or untransfected parental 293 cells in PBS. Tumors were allowed to grow and were measured with digital calipers, over a period of three weeks. Experiments were terminated after three weeks when the first animal reached the maximum size allowed by the Institutional Ethics Committee. The tumor size was calculated as the width x length x 0.6 x (width x length)/2.

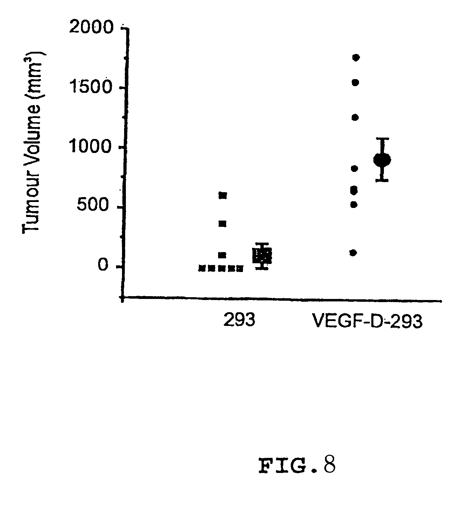

Figure 8 shows the results of the analysis of tumors in SCID mice resulting from injection of untransfected parental 293 cells (designated "293") or 293 cells transfected with the construct encoding VEGF-D-FULL-N-FLAG (designated "VEGF-D-293"). There is significant difference between the tumors derived from the 293-VEGF-D-FULL-N-FLAG cells and those derived from the untransfected 293 cells. After three weeks the mean tumor size of the 293-VEGF-D-FULL-N-FLAG group was 937± 55.5 mm³ (mean ± SD, n = 8) compared to 136± 230 mm³ for the untransfected 293 cells (n = 8). Interestingly, tumors generated from 293 cells transfected with a construct encoding VEGF-DΔNΔC were not significantly different in size, 50 ± 76 mm³ (n = 7), to those from the untransfected 293 cells.

In addition, the macroscopic appearance of tumors derived from the untransfected 293 cells was one of a pale white surface, compared to the tumors derived from the 293-VEGF-D-FULL-N-FLAG cells which had a bloody appearance, with the presence of blood vessels apparent throughout the tumor.

Also, sections were analyzed by immunohistochemistry with an anti-PECAM monoclonal antibody (Pharmingen, San Diego, CA), a marker of endothelial cells. Sections of tumors generated with 293-VEGF-D-FULL-N-FLAG cells demonstrated a marked increase in PECAM expression compared to the tumors generated with untransfected parental 293 cells. This analysis confirms the much greater abundance of blood vessels in the tumors expressing unprocessed full-length VEGF-D.

This experiment indicates that the unprocessed form of VEGF-D is capable of inducing tumor angiogenesis and the growth of a solid tumor *in vivo*. Interestingly, the tumors derived from cells expressing the mature, fully processed form of VEGF-D showed no increase in growth compared to the untransfected 293 parental cells. This indicates the importance of the propeptides (N-pro and C-pro) in VEGF-D for the correct localization or function of the VHD of VEGF-D. An explanation for this result is that the propeptides are involved in matrix association and only when VEGF-D is positioned correctly on the extracellular matrix or cell surface heparin sulphate proteoglycans is the growth factor able to induce angiogenesis and/or lymphangiogenesis. An alternative explanation is that the propeptides increase the half-life of the VEGF-D VHD *in vivo*.

### Example 8 VEGF-D induction of tumor angiogenesis

To determine whether VEGF-D plays a role in tumor angiogenesis, 293EBNA cell lines expressing VEGF or VEGF-D were generated. 293EBNA cells normally do not express detectable levels of VEGF, VEGF-C, or VEGF-D, the ligands that activate VEGFR-2 and/or VEGFR-3 (Stacker, S.A., *et al*., *Growth Factors* **17**: 1-11 (1999)). 293EBNA cells produce slow growing and poorly vascularized epithelioid-like tumors in immunodeficient mice. Western-blot analysis of conditioned medium from the generated 293EBNA cell lines *in vitro* showed that the mature forms of the active growth factors were secreted.

Six to twenty-one week old female SCID or SCID/nod mice (Animal Resources Center, Canning Vale, Australia; Austin Research Institute, Australia; and Walter and Eliza Hall Institute for Medical Research, Australia) were placed in groups of 6 to 10 mice and injected subcutaneously in the mammary fat pad with cell lines expressing VEGF-293, VEGF-D-293, or control 293 cell lines at a concentration of 2.0-2.5 x 10⁷ in culture medium. Tumor growth and morphology were analyzed over 35 days. Tumors were measured with digital calipers and tumor volume was calculated by the formula: volume = length x width² x 0.52. Three to five weeks after injection with cell lines the mice were euthanized and the tumors were removed for examination. VEGF-D-293 tumors and 293 tumors were excised post mortem on day 25 and weighed.

VEGF-293 cells produced tumors with an increased growth rate compared with control 293 cells. The VEGF-293 tumors were highly vascularized with extensive edema, consistent with VEGF being a potent tumor angiogenesis factor and an inducer of vascular permeability. VEGF-D-293 cells also showed enhanced growth *in vivo* and the tumors were highly vascularized compared with control 293 tumors but showed no evidence, overtly or microscopically, of edema.

Tumor growth arising from injection of VEGF-D-293 cells was blocked by twice weekly intraperitoneal injections of monoclonal antibody VD1, an antibody specific for the bioactive region of VEGF-D that blocks binding of VEGF-D to VEGFR-2 and VEGFR-3. However, tumor growth was unaffected by treatment with a control, isotype-matched antibody.

Treatment with the VD1 antibody reduced the abundance of vessels in the tumors as assessed by immunohistochemistry for the endothelial cell marker PECAM-1. Western blotting demonstrated the expression of VEGF-D and VEGF in VEGF-D-293 and VEGF-293 tumors, respectively, and also that VEGF was not upregulated in VEGF-D-293 tumors. Analysis of tumor weights post mortem demonstrated a significant difference between the VEGF-D-293 tumors (0.49±0.22 g, n=7; mean ± SD) and the control 293 tumors (0.123±0.118 g, n=9, p=0.01).

### Example 9 VEGF-D induction of tumor lymphangiogenesis

Because metastasis to local lymph nodes via the lymphatic vessels is a common step in the spread of solid tumors, experiments were conducted to determine if VEGF-D induced tumor lymphangiogenesis, or if expression of VEGF-D in tumor cells led to spread of the tumor to lymph nodes.

To analyze the role of VEGF-D in tumor spread, VEGF-D-293 tumors were induced in SCID/NOD mice (Animal Resources Center, Canning Vale, Australia; Austin Research Institute, Australia; and Walter and Eliza Hall Institute for Medical Research, Australia). Post-mortem analysis revealed that animals with VEGF-D-293 tumors had developed metastatic lesions in either the lateral axillary lymph node and/or superficial inguinal nodes in 14 of 23 animals compared with 0 of 16 animals for VEGF-293 tumors and 0 of 14 animals for 293 tumors. In some cases, the spread of metastatic tumor cells from the primary tumor in SCID/NOD mice was evident as a trail of tumor cells in the lymphatics of the skin between the primary tumors and the lateral axillary node.

Treatment of mice harboring VEGF-D-293 tumors with the VD1 monoclonal antibody (Table 1) blocked the metastatic spread to lymph nodes. None of the 7 mice treated over 25 days with VD1 exhibited lymphatic spread, whereas 6 of 10 mice treated with a control isotype-matched monoclonal antibody exhibited lymphatic spread. These results indicate that VEGF-D can promote the metastatic spread of these tumors via the lymphatics.

**Table 1:**

| Metastatic spread of tumors in SCID/NOC mice | | |
|---|---|---|
| Tumor line | Number of mice with primary tumors | Number of mice with spread to local lymph nodes |
| VEGF-D-293 | 23 | 14(61%) |
| VEGF-D-293 (VD1-treated)^{a} | 7 | 0 |
| VEGF-D-293 (LMM774-treated)^{b} | 10 | 6(60%) |

| | | |
|---|---|---|
| ^{a} Purified monoclonal antibodies were injected twice weekly over the course of the experiment, starting 1 day after injection of the tumor cells. VD1 is a neutralizing monoclonal antibody against VEGF-D. | | |
| ^{b} LMM774 is an isotype-matched control monoclonal antibody that does not bind VEGF-D. | | |

The data show that expression of VEGF-D can promote metastatic spread of tumor cells through the lymphatic network. VEGF-D induced formation of lymphatic vessels in the tumors, as detected by immunohistochemistry for the lymphatic-specific marker LYVE-1, presumably through the lymphatic receptor VEGFR-3, although activation of VEGFR-3-VEGFR-2-heterodimers cannot be excluded. The expression of lymphangiogenic factors alone is sufficient to induce the formation of lymphatic vessels in the center of a tumor and to facilitate the metastatic spread to the lymph nodes.

VEGF-D was localized to tumor cells and the endothelium of vessels in lung and breast cancers (see Examples 5-6).

### Example 10 Variance in tumor characteristics induced by different forms of VEGF-D

In addition to the determination of the role of VEGF-D in tumor angiogenesis and lymphangiogenesis, the methods of Example 10 and 11 were used to produce and evaluate tumors expressing different forms of VEGF-D which represent the cleavage of the N, C, and both N and C terminal propeptides. The cell lines injected into the mice were 293EBNA, VEGF-D-293, VEGF-DΔNΔC-293, VEGF-DΔC-293 (cells expressing VEGF-D lacking the C-terminal propeptide), and VEGF-DΔN-293 (cells expressing VEGF-D lacking the N-terminal propeptide).

The tumors produced by the VEGF-DΔN cells grew more rapidly than the tumors produced by control cells. Upon morphological examination the tumors were red in appearance and contained a significant vascular reaction, including a substantial fluid component not seen in the control tumors. The tumors produced by the VEGF-DΔN cells had significant differences in growth and morphological characteristics than the control tumors.

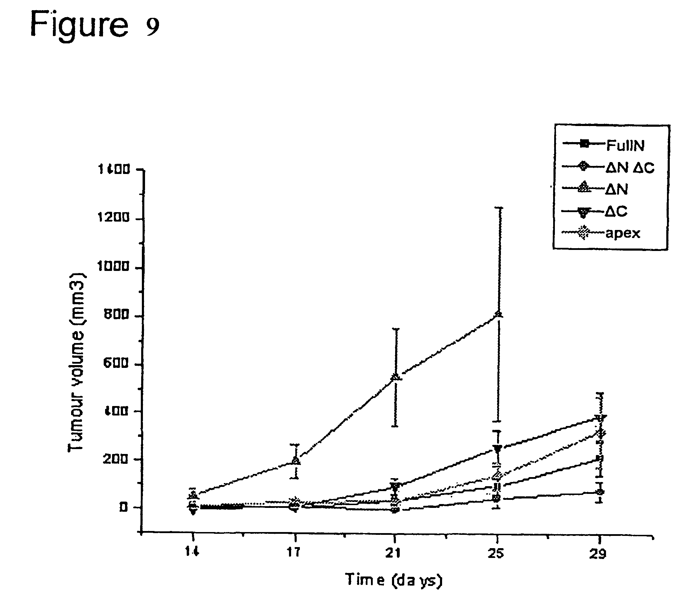

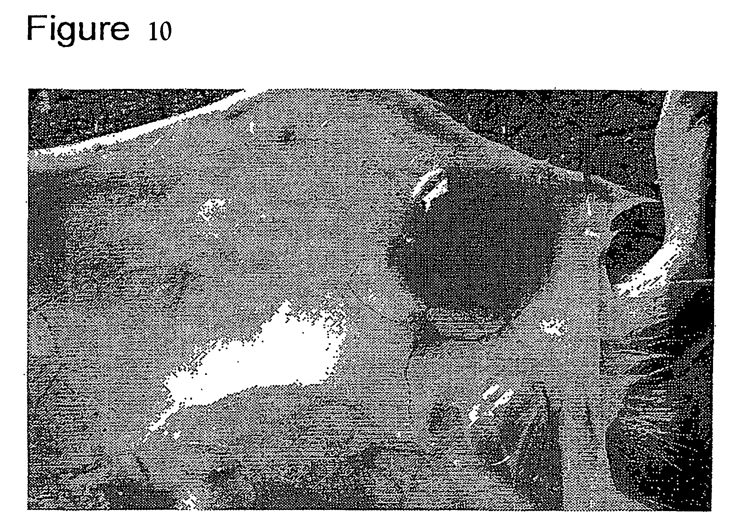

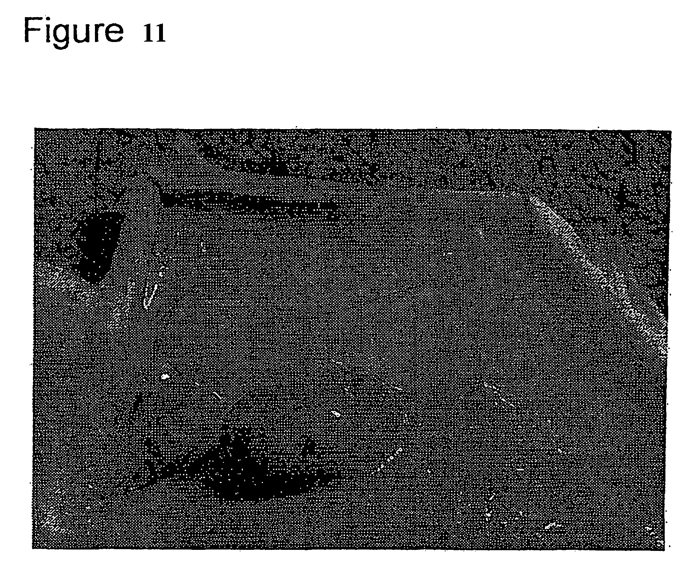

The graph of Figure 9 shows the increased rate of growth in tumors from the VEGF-DΔN cells. The tendency toward fluid accumulation in the tumors produced by the VEGF-DΔN cells can be seen in Figure 10, a photograph of such a tumor. This can be contrasted with the photograph of Figure 11 which depicts a normal tumor such as that produced by the control cells.

The tumors produced by the VEGF-DΔC cells grew in a similar fashion to the control cells and did not exhibit excess fluid formation.

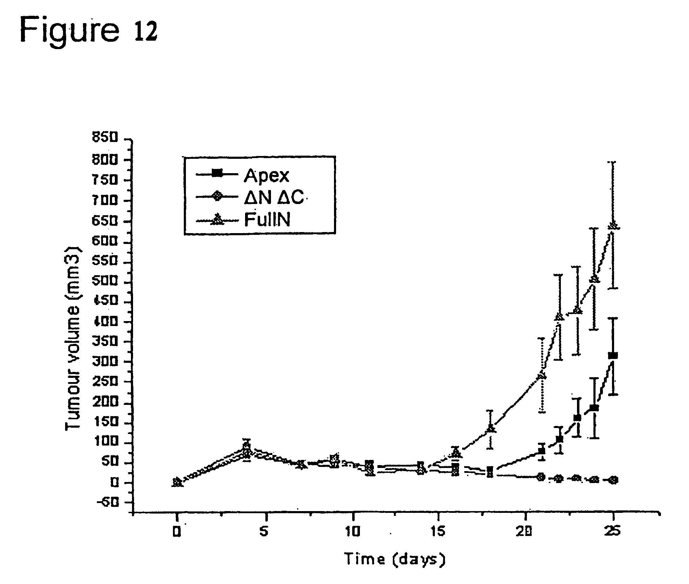

The tumors produced by the VEGF-DΔNΔC cells grew very slowly compared to the control tumors. The VEGF-DΔNΔC tumors formed in about 70 days as compared to an average 30-35 days for the control tumors and 20-25 days for the VEGF-DΔN tumors. Examination of these tumors showed that they had a reduced vascular response, having fewer blood vessels than control tumors by PECAM-1 staining. The tumors developed lymphatic networks as shown by LYVE-1 staining and induced formation of lymphatic metastases. The graph of Figure 12 shows the decreased rate of growth in tumors from the VEGF-DΔNΔC cells.

Similar patterns of staining to those seen in the melanomas were observed in squamous cell carcinoma of the lung and breast ductal carcinoma *in situ* (BDCIS) as VEGF-D was detected in tumor cells and on vessels nearby. Vessels near the tumor-filled ducts in BDCIS and near the islands of tumor cells in lung carcinoma were also positive for VEGFR-2, again suggesting this ligand and receptor may contribute to the control of tumor angiogenesis in a paracrine fashion.

These results also indicate that VEGF-D may play a role in stimulating the growth of lymphatic vessels in the vicinity of malignant melanoma as vessels positive for VEGFR-3, a receptor for VEGF-D expressed on lymphatic endothelium in normal adult tissues, were also positive for VEGF-D. Similar staining patterns were seen in BDCIS as some of the VEGF-D positive vessels surrounding the tumor-filled ducts were also positive for VEGFR-3. Signaling via VEGFR-3 is thought to be important for lymphangiogenesis (Taipale, J. *et al*., *Curr Top Microbiol Immunol* **237**: 85-96, 1999), although this receptor can be up-regulated on blood vessel capillaries in cancer (Valtola, R. *et al*., *Am*. *J. Path.* **154**: 1381-1390, 1999). Therefore the paracrine regulatory system consisting of VEGF-D and VEGFR-3 could stimulate both lymphangiogenesis and angiogenesis in cancer. Accordingly, the route by which a tumor metastasizes may be determined, in part, by its capacity to induce angiogenesis and/or lymphangiogenesis. If so, the expression by tumor cells of soluble growth factors which are purely angiogenic (e.g. VEGF) as opposed to those which may also induce lymphangiogenesis (e.g. VEGF-D) could be an important determinant of the route of metastatic spread.

## Claims

1. The use of a VEGF-D antagonist which prevents binding of VEGF-D to its corresponding receptor for the manufacture of a medicament for use in the treatment of a neoplastic disease **characterised in that** said neoplastic disease is selected from the group consisting of: breast ductal carcinoma, squamous cell carcinoma, prostate cancer and endometrial cancer.

2. Use according to claim 1, wherein said VEGF-D antagonist is co-administered with a cytotoxic agent.

3. Use according to claim 1 or 2, wherein said antagonist further comprises at least one pharmaceutical carrier or adjuvant.

4. Use according to any of claims 1-3, wherein said antagonist is a monoclonal antibody which specifically binds VEGF-D and blocks VEGF-D binding to VEGF Receptor-2 or VEGF Receptor 3.

5. Use according to claim 4, wherein said antibody binds to the VEGF homology domain of VEGF-D.

## Patentansprüche

1. Verwendung eines VEGF-D-Antagonisten, der die Bindung von VEGF-D an seinen entsprechenden Rezeptor verhindert, zur Herstellung eines Medikaments zur Verwendung bei der Behandlung einer neoplastischen Erkrankung, **dadurch gekennzeichnet, daß** die neoplastische Erkrankung ausgewählt ist aus der Gruppe bestehend aus: duktalem Brustkarzinom, squamösem Zellkarzinom, Prostatakrebs und Endometriumkrebs.

2. Verwendung nach Anspruch 1, wobei der VEGF-D-Antagonist zusammen mit einem zytotoxischen Mittel verabreicht wird.

3. Verwendung nach Anspruch 1 oder 2, wobei der Antagonist ferner wenigstens einen pharmazeutischen Träger- oder Hilfsstoff umfaßt.

4. Verwendung nach irgendeinem der Ansprüche 1-3, wobei der Antagonist ein monoklonaler Antikörper ist, der VEGF-D spezifisch bindet und die Bindung von VEGF-D an VEGF-Rezeptor 2 oder VEGF-Rezeptor 3 blockiert.

5. Verwendung nach Anspruch 4, wobei der Antikörper an die VEGF-Homologiedomäne von VEGF-D bindet.

## Revendications

1. Utilisation d'un antagoniste VEGF-D qui prévient la liaison de VEGF-D à son récepteur correspondant pour la fabrication d'un médicament pour l'utilisation lors du traitement d'une maladie néoplasique, **caractérisé en ce que** ladite maladie néoplasique est choisie dans le groupe consistant en : carcinome canalaire de poitrine, carcinome cellulaire squameux, cancer de la prostate et cancer de l'endomètre.

2. Utilisation selon la revendication 1, **caractérisée en ce que** ledit antagoniste VEGF-D est co-administré avec un agent cytotoxique.

3. Utilisation selon la revendication 1 ou 2, **caractérisée en ce que** ledit antagoniste comprend au moins un adjuvant ou porteur pharmaceutique.

4. Utilisation selon l'une des revendication 1 à 3, **caractérisée en ce que** ledit antagoniste est un anticorps monoclonal qui se lie spécifiquement à VEGF-D et bloque la liaison de VEGF-D au récepteur-2 VEGF ou récepteur-3 VEGF.

5. Utilisation selon la revendication 4, **caractérisée en ce que** ledit anticorps se lie au domaine homologue VEGF de VEGF-D.
